Europäisches Patentamt

European Patent Office

Office européen des brevets

(11) Publication number: **0 248 167**
**A2**

# EUROPEAN PATENT APPLICATION

(21) Application number: 87105121.5

(51) Int. Cl.⁴: **C12N 15/00** , C12P 21/02

(22) Date of filing: 07.04.87

(30) Priority: 07.04.86 JP 78315/86

(43) Date of publication of application:
09.12.87 Bulletin 87/50

(84) Designated Contracting States:
CH DE ES FR GB LI

(71) Applicant: **THE GREEN CROSS CORPORATION**
**15-1, Imabashi-1-chome Higashi-ku**
**Osaka-shi**
**Osaka 541(JP)**

(72) Inventor: **Mukai, Hiromichi**
**5-2-103, Fujisaka Higashi-cho 2-chome**
**Hirakata-shi Osaka(JP)**
Inventor: **Tsujikawa, Muneo**
**21-302, Kita Funabashi-cho**
**Hirakata-shi Osaka(JP)**
Inventor: **Horii, Hajime**
**2-18-3, Segawa**
**Minoo-shi Osaka(JP)**
Inventor: **Kawabe, Haruhide**
**29-14, Yamada Nishi 3-chome**
**Suita-shi Osaka(JP)**
Inventor: **Arimura, Hirofumi**
**18-1-401, Uenozaka 2-chome**
**Toyonaka-shi Osaka(JP)**
Inventor: **Nishida, Masayuki**
**12-1, Kayogaoka 2-chome**
**Nagaokakyo-shi Kyoto(JP)**
Inventor: **Suyama, Tadakazu**
**3-7, Matsuigaoka 4-chome Tanabe-cho**
**Tsuzuki-gun Kyoto(JP)**

(74) Representative: **Hansen, Bernd, Dr.rer.nat. et**
**al**
**Hoffmann, Eitle & Partner Patentanwälte**
**Arabellastrasse 4**
**D-8000 München 81(DE)**

(54) Method of producing PRE S-HBsAg and the PRE S-HBsAg.

(57) A method of producing Pre S-HBsAg comprising
(A) culturing <u>Saccharomyces</u> <u>cerevisiae</u> which has been transformed with an expression vector comprising pBR322 having inserted therein in sequence, a substantial part of the 164-base-pair region of the yeast glyceraldehyde-3-phosphate dehydrogenase (GAP-DH) promoter upstream from the initiation codon for the GAP-DH portion, and the Pre s-HBsAg gene containing at least a late part of the Pre S region of HB virus, wherein said substantial part of the 164-base-pair region beginning with the -164th base pair upstream from said initiation codon and
(B) obtaining Pre S-HBsAg for the culture supernatant, as well as the said Pre S-HBsAg.

## METHOD OF PRODUCING PRE S-HBsAg AND THE PRE S-HBsAg

### FIELD OF THE INVENTION

The present invention relates to a method of producing Pre S-HBsAg (HBsAg including the polypeptide portion encoded by at least a late part of the Pre S region) using an improved yeast promoter.

### BACKGROUND OF THE INVENTION

The expression of heterologous genes in yeast, e.g., HBsAg and IFN-α has been achieved. The mechanism of yeast promoter control is generally a positive control, with some exceptions such as in the case of glucose inhibition. The presence of an upstream activator sequence (UAS) functioning in the cis mode at a site several hundred bases upstream from the translation initiation site has been suggested (Guarente L. et al, Cell, 36:799 (1984)). The UAS is believed to serve as a promoter-side regulatory factor involved in such positive control. Furthermore, it has been shown that replacement of this UAS with another UAS leads to the release of the promoter from the original control system and the placement of the promoter under the control involving the new substitute UAS (Guarente, L. et al, Proc. Natl. Acad. Sci. USA, 79:7410 (1982); Guarente, L. et al, Cell, 36:503 (1984); Struhl, K., Proc. Natl. Acad. Sci. USA, 81:7865 (1984). Therefore, one way to improve yeast pro moters is to modify the control system by replacement of the UAS or for another UAS by removing the control system by DNA deletion or miniaturization of the promoter.

The glyceraldehyde-3-phosphate dehydrogenase gene (GAP-DH) promoter is one of the yeast promoters which has been used in constructing hepatitis B virus surface antigen (HBsAg)-providing plasmids (Bitter, G.A. & Egan, K. M., Gene , 32:263-274 (1984).

It is known that the HBsAg proteins include not only the major polypeptides P24 and P27 (P24 plus sugar chain) but also P33 and P37 (P33 plus sugar chain). According to Machida el al, Gastroenterology, 85:268 (1983), the amino acid sequence of P33 is composed of the 226 amino acid residues of P24 and an additional 55 amino acid residues encoded by the Pre S region.

The mechanism of invasion of HB virus into hepatocytes is believed to involve the polyalbumin (polymerized albumin) receptor on HBV, polyalbumin and the polyalbumin receptor on the hepatocyte. Since the polyalbumin receptor is encoded by the Pre S region of HB virus, HBsAg having the Pre S region gene product, i.e., Pre S-HBsAg, is believed to induce increased antibody production in humans as compared with HBsAg having no Pre S region polypeptide. In fact, it has been reported that Pre S-HBaSg can cause increased antibody production in vitro (Neurath, A.R. et al. Science , 224:392-395 (1984)).

### SUMMARY OF THE INVENTION

The present invention has been accomplished based on the finding that the Pre S region polypeptide-containing HBsAg (Pre S-HBsAg) can be produced by using the GAP-DH promoter having a deletion therein upstream from -164 bp.

Thus the invention provides a method of producing Pre S-HBsAg which used, as a promoter, the DNA sequence contained in the GAP-DH promoter and included in the region from -164 bp down to the initiation codon for the GAP-DH protein.

### BRIEF DESCRIPTION OF THE DRAWINGS

Fig. 1 shows the base sequence of a miniaturized GAP-DH promoter (from -164 bp to -25 bp);

Fig. 2 (A) and Fig. 2 (B) shows a scheme for preparing pGG5 which carries the GAP-DH promoter;

Fig. 3 shows a scheme for preparing pGL5 and pGL6;

Fig. 4 shows a scheme for preparing the HBsAg production plasmid pGG6 which carries the miniaturized GAP-DH promoter;

Fig. 5 shows a scheme for preparing the Pre S-HBsAg production plasmid pGG53 which carries the GAP-DH promoter;

Fig. 6 shows a construction scheme for preparing the Pre S-HBsAg production plasmid pGG63 which carries the miniaturized GAP-DH promoter;

Fig. 7 shows the restriction enzyme map of the Pre S-HBsAg gene used in the invention;

Fig. 8 shows a scheme for preparing the Pre S-HBsAg production plasmid pGG62.

In the figures:

1 = ··· GAP-DH gene,

2 = ··· GAP-DH promoter,

3    GAP-DH promoter DNA fragment,

4 = ··· Terminator for GAP-DH gene,

5    DNA fragment containing 4,

6    1.3 kb DNA fragment containing HBsAg gene,

7 = ··· HBsAg gene, and

8 = ··· Pre S gene.

## DETAILED DESCRIPTION OF THE INVENTION

The improved GAP-DH promoter according to the invention is prepared in the following manner.

The GAP-DH promoter to be used in the practice of the invention is known and its base sequence has been disclosed (Holland, J.P. and Holland, M.J., J. Biol. Chem. , 254 (19):9839-98945 (1979)). Therefore, the starting material for the improvement according to the invention can be prepared by a known method. For instance, it can be prepared easily from yeast chromosomal DNA as described hereinafter in Reference Example 1.

The GAP-DH promoter gene is miniaturized, either after isolation or in the plasmid-borne form, with a specific restriction enzyme. The deletion is effected upstream from the initiation codon for the GAP-DH protein, most preferably at about -164 bp. Suitable examples of the restriction enzyme include Xmnl and Taql. The miniaturization is finished by further cleavage with a restriction enzyme at about -25 bp upstream from said initiation codon.

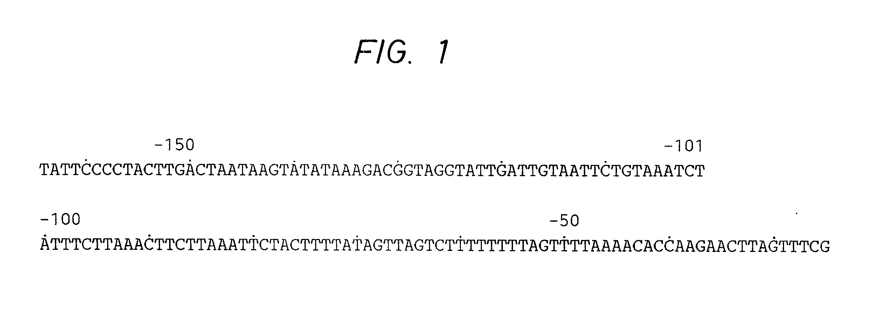

The DNA sequence of such a miniaturized promoter was determined by the Maxam-Gilbert method and the base sequence is shown in Fig. 1. This base sequence, although in agreement with the corresponding part of the known sequence, is very small in size.

This miniaturized GAP-DH promoter is inserted into a plasmid by known procedures using restriction enzymes and ligase.

A recombination plasmid is produced by insertion of a gene coding for Pre S-HBsAg downstream from the thus-miniaturized GAP-DH promoter. The recombinant plasmid is used to transform a yeast strain. In this way, Pre S-HBsAg can be produced efficiently by causing expression of said gene.

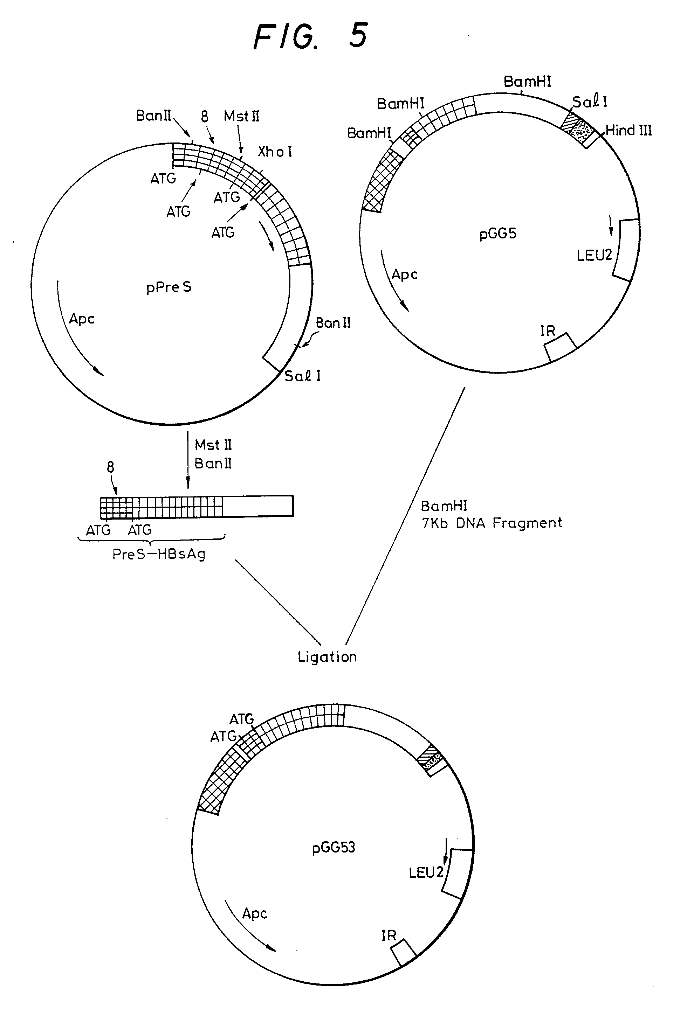

The DNA sequence of Pre S is known and plasmids carrying Pre S are also known (Molecular Cloning, Cold Spring Harbor Laboratory (1982)). For example, the sequence shown is known as the full length, DNA sequence of Pre S. In the examples of present invention, the plasmid pPre S (Fig. 5) commercially available from Biogen was employed. The use of the entire length of the Pre S region is not always necessary. The fragment resulting from cleavage at the second or third ATG site is sufficient for the purpose of the invention and in fact is preferable.

3

# DNA and Amino Acid Sequence of Pre S

1
Met Gly Gln Asn Leu Ser Thr Ser Asn
ATG GGG CAG AAT CTT TCC ACC AGC AAT

10
Pro Leu Gly Phe Phe Pro Asp His Gln Leu
CCT CTG GGA TTC TTT CCC GAC CAC CAG TTG

20
Asp Pro Ala Phe Arg Ala Asn Thr Asn Asn
GAT CCA GCC TTC AGA GCA AAC ACC AAC AAT

30
Pro Asp Trp Asp Phe Asn Pro Asn Lys Asp
CCA GAT TGG GAC TTC AAT CCC AAC AAG GAC

40
Thr Trp Pro Asp Ala Asn Lys Val Gly Ala
ACC TGG CCA GAC GCC AAC AAG GTA GGA ACT

50
Gly Ala Phe Gly Leu Gly Phe Thr Pro Pro
GGA GCA TTC GGG CTA GGG TTC ACC CCA CCG

60
His Gly Cly Leu Leu Gly Trp Ser Pro Gln
CAC GGA GGC CTT TTG GGG TGG AGC CCT CAG

70
Aka Gln Gly IIe Met Gln Thr Leu Pro Ala
GCT CAG GGC ATA ATG CAA ACC TTG CCA GCA

80
Asn Pro Pro Pro Ala Ser Thr Asn Arg Gln
AAT CCG CCT CCT GCC TCT ACC AAT CGC CAG

90
Ser Gly Arg Arg Pro Thr Pro Leu Ser Pro
TCA GGA CGG CAG CCT ACC CCG CTG TCT CCA

100
Pro Leu Arg Thr Thr His Pro Gln Ala
CCT CTG AGA ACC ACT CAT CCT CAG GCC

110
Met His Trp Asn Ser Thr Thr Phe His Gln
ATG CAC TGG AAC TCC ACA ACC TTC CAC CAA

120
Thr Leu Gln Asp Pro Arg Val Arg Gly Leu
ACT CTG CAA GAT CCC AGA GTR AGA GGC CIG

130
Tyr Phe Pro Ala Gly Gly Ser Ser Ser Gly
TAT TCC CCT GCT GGT GGC TCC AGT TCA GGG

140
Thr Val Asn Pro Val Pro Thr Thr Thr Ser
ACA GTA AAC CCT GTT CCG ACT ACT ACC TCT

150
Pro Ile Ser Ser Ile Phe Ser Arg Ile Gly
CCC ATA TCG TAC ATC TTC TCG AGG ATT GGG

160
Asp Pro Ala Leu Asn
GAC CCT GCG CTG AAC

0 248 167

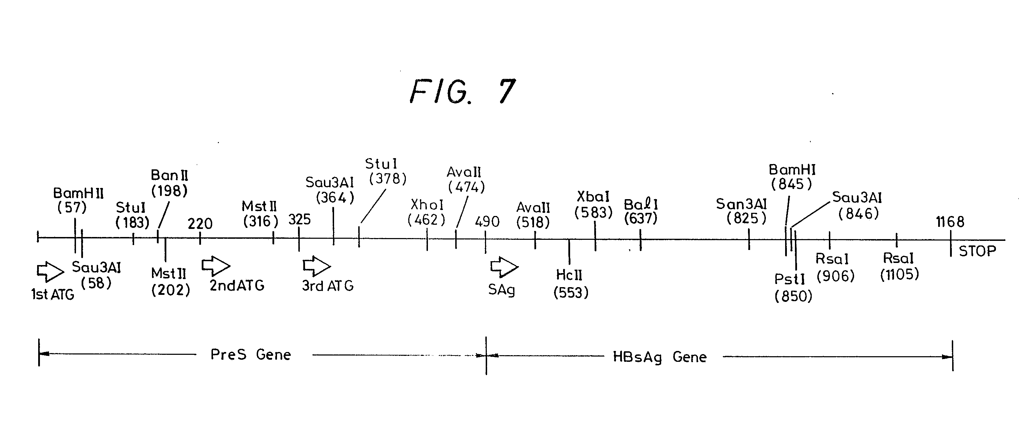

The DNA sequence of the HBsAg gene is also known and plasmids carrying said gene are known. The Pre S-HBsAg gene has the restriction enzyme map shown in Fig. 7. In the examples of the present invention, the plasmid pPre S (Fig. 5) commercially available from Biogen was used.

While HBsAg includes the subtypes adw (Valenzucala et al; Nature, 280:815-819 (1979), ayw (Charnay et al, Proc. Natl. Acad. Sci. U.S.A. , 76:2222-2226 (1979), ad/yw (Pasek et al, Nature, 282:575-579 (1979), etc., HBsAg subtype ad/yw was employed in the example of the present invention.

The expression vector to be used in the practice of the invention is a vector containing a yeast gene, or Escherichia coli gene and further the miniaturized yeast GAP-DH promoter. The vector carries marker genes for the Escherichia coli transformation and yeast transformation, for example, and ampicillin resistance gene, 2-isopropyl malate dehydrogenase gene, etc.

The yeast strain to be transformed is suitably a mutant with a mutation complementary to the selection marker gene borne by the plasmid to be inserted, for example, the leucine-requiring mutant Saccharomyces cerevisiae AH22 (a, his4, leu2, can1) deposited at American Type Culture Collection, 12301 Parklawn Drive, Rockville, Maryland 20852-1776 under ATCC No. 38626 (G. Fink, Proc. Natl. Acad. Sci. USA, 75, 1929 (1978). The transformed yeast obtained is culti vated on a known medium suited for the host, for example, YNB liquid medium comprising 0.7% (w/v) Yeast Nitrogen Base (Difco), 2% (w/v) glucose. The cultivation is carried out generally at 15-32°C for 20-50 hours, with aeration and/or stirring, if desired. After cultivation, the cells are collected by a known method, for example, by centrifugation and then disrupted, for example, by suspending them in an appropriate buffer solution and subjecting the suspension to sonication. Thereafter, the supernatant is recovered. This supernatant contains the desired product Pre S-HBsAg and this desired substance is purified, for example, by affinity chromatography using a column with a monoclonal antibody immobilized thereon or by affinity chromatography based on hydrophobic groups.

A large number of techniques, reaction methods and analytical methods are well known in the art relative to the practice of the invention. Unless otherwise specified, all of the enzymes are commercially available, for example, from Takara Shuzo, Japan; New England Biolabs (NEB), Massachusetts, U.S.A.; Amersham, Great Britain; and Bethesda Research Laboratories (BRL), Maryland, U.S.A.

Unless otherwise specified, the buffer and reaction conditions for each enzymatic reaction were as recommended by the manufacturer of the enzyme used.

Transformation of Escherichia coli utilizing a phage, transformation of Escherichia coli with a plasmid, plaque hybridization, electrophoresis and recovery of DNA from the gel were carried out as described in Molecular Cloning, supra. Yeast transformation was conducted as described in Methods in Yeast Genetics, Cold Spring Harbor Laboratory (1981).

Suitable examples of Escherichiacoli strains which can be used in the present invention include E. coli HB101 available from Takara Shuzo (D. Hanahan, J. Mol. Biol., 166, 557 (1983)), E. coli JM109 available from Takara Shuzo (J. Messing, Gene, 33, 103 (1985)), E. coli RRI available from BRL (Maryland, U.S.A.) (D. Hanahan, J. Mol. Biol., 166, 557 (1983)), and those disclosed in Molecular Cloning, supra.

The production system comprising yeast carrying the thus-obtained miniaturized GAP-DH promoter plus the Pre S-HBsAg gene enables efficient expression of the Pre S-HBsAg protein. Through the use of such miniaturized GAP-DH promoter that has been so far unknown, the procedure of DNA recombination is simplified. Furthermore, since it is a miniaturized one, the promoter according to the present invention can be synthesized more easily. Thus, production of the promoter can be performed with increased productivity and the promoter construction can be expected to become more productive.

Pre S-HBsAg produced in yeast using the above minia turized promoter or an improvement thereof (for example, promoters comprising a miniaturized promoter connected at its upstream end with the upstream portion of another promoter such as Pho 5, PGK, etc., containing UAS or with an SV40 enhancer) can be anticipated to have higher antigenicity in humans than the conventional HBsAg species produced in yeast. Hence, the resulting Pre S-HBsAg can be expected to serve as a more effective hepatitis B vaccine than hepatitis B vaccines which use HBsAg.

The following reference examples and working examples illustrate the invention in more detail but are by no means limitative of the invention.

REFERENCE EXAMPLE 1

Cloning of the yeast GAP-DH gene

DNA having the yeast GAP-DH gene was prepared from yeast chromosomal DNA in the following manner.

The chromosomal DNA was prepared from Saccharomyces cerevisiae GRF18 ($\alpha$, leu, trp, his, met) obtained from Biogen S.A. (Erhart, E. & Hollenberg, C.P., J. Bacteriol. , 156, 625 (1983)) by the method of C.R. Cryer et al, Methods in Cell Biology, Vol. 18, Chapter 3, page 39, Academic Press, New York (1975).

A 20-$\mu$g portion of this chromosomal DNA was completely digested with 10 units (U) of the restriction enzyme Hin dIII (Takara Shuzo; the same shall apply hereinafter) and joined with 1 $\mu$g of lamba phage charon 28 (B1007, KH54, N1N5) DNA also completely digested with 1 U of HindIII. For the joining reaction, T4 DNA ligase (Takara Shuzo; the same shall apply hereinafter) was used. The reaction was carried out overnight at 16°C following the recommended procedure. The same method was also used in the DNA joining or ligation reactions to be mentioned hereinafter. The ligated product DNA was packaged using an in vitro packaging kit (Amersham) and then used to infect the Escherichia coli strain LE392 (F⁻, hsdR514, (r$_k$⁻, m$_k$⁻), supE44, supF58, lacY1, galK2, galT22, metB1, trpR55, $\lambda$⁻) obtained from Stratagene (San Diego, U.S.A.) (Murray, N.E., Brammer, W.J. & Murray, K., Mol. Gen. Genet. , 150, 53 (1977)). In this manner, 40,000 plaques were obtained. The packaging was performed as recommended by Amersham and the infection of Escherichia coli was conducted as described in Molecular Cloning, supra.

These 40,000 plaques were screened by immobilizing them on a nitrocellulose filter and then hybridizing them with $^{32}$p-labeled synthetic DNA (plaque hybridiation). The synthetic DNA had the base sequence corresponding to 19th to 33rd from the first ATG of the GAP-DH gene (AACGGTTTCGGTAGA) reported by J.P. Holland et al (J. Biol. Chem., 254, 19, 9839 (1979)). The plaque hybridization was conducted as described in Molecular Cloning , supra. As a result, two phages capable of strong hybridization and agreeing in restriction enzyme mapping were obtained.

A yeast chromosome-derived 2.1 kb DNA fragment was prepared by completely digesting 10 $\mu$g of this phage DNA with 2 U of HindIII. The 2.1 kb DNA fragment was recovered by subjecting the completely HindIII-digested DNA to electrophoresis using low-melting agarose (BRL) in a concentration of generally 0.5 to 1.5%, preferably 0.7 to 1%, excising the 2.1 kg DNA fragment gel segment, extracting the DNA with phenol after 10-minute of heat treatment at 65°C as recommended by BRL and precipitating the DNA from the aqueous layer with ethanol. The DNA fragment recoveries described hereinafter were always carried out by the above method.

A 1-$\mu$g portion of this 2.1 kb HindIII DNA fragment was joined to 1 $\mu$g of the pBR322 DNA, a typical Escherichia coli-derived plasmid. which had been completely digested with 1 U of HindIII using 5 U of T4 DNA ligase. The ligation product was used to transform the Escherichia coli strain HB101 (F⁻. hsdS20(r$_B$⁻, m$_B$⁻). recA13. ara -14. proA2. lacY1, galK2, rpsL20(Sm$^r$), xyi-5. mtl -1, supE44. $\lambda$⁻) (Takara Shuzo) and cloning was performed. The transformation of the Ewcherichia coli strain with the plasmid was performed as described in Molecular Cloning, supra. The plasmid obtained by the above cloning was named pGAP301.

REFERENCE EXAMPLE 2

Construction of HBsAg expression vector

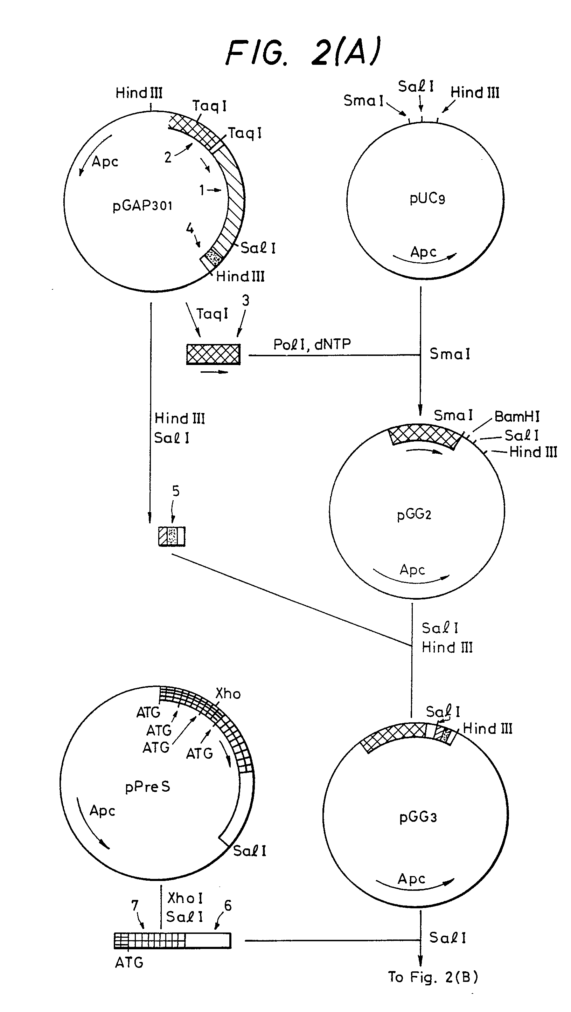

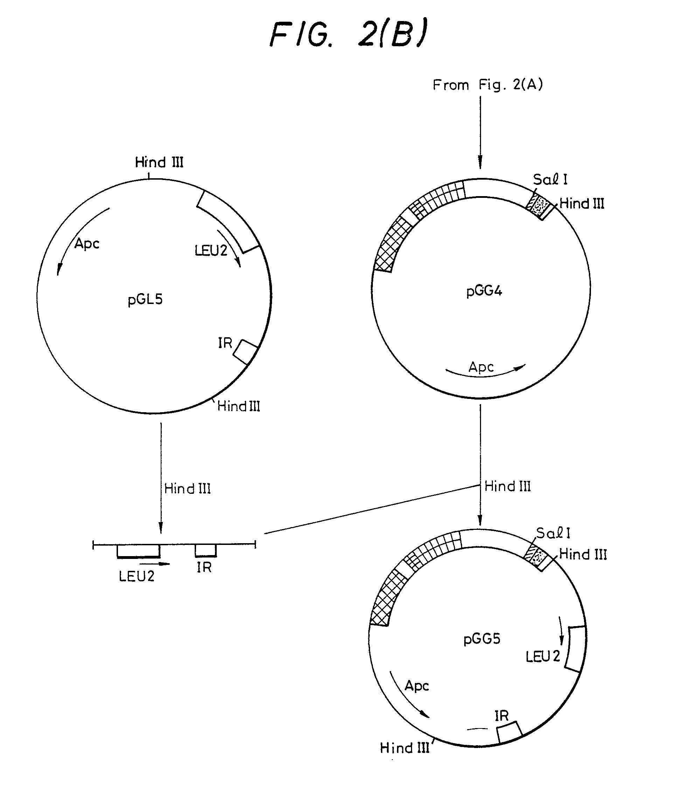

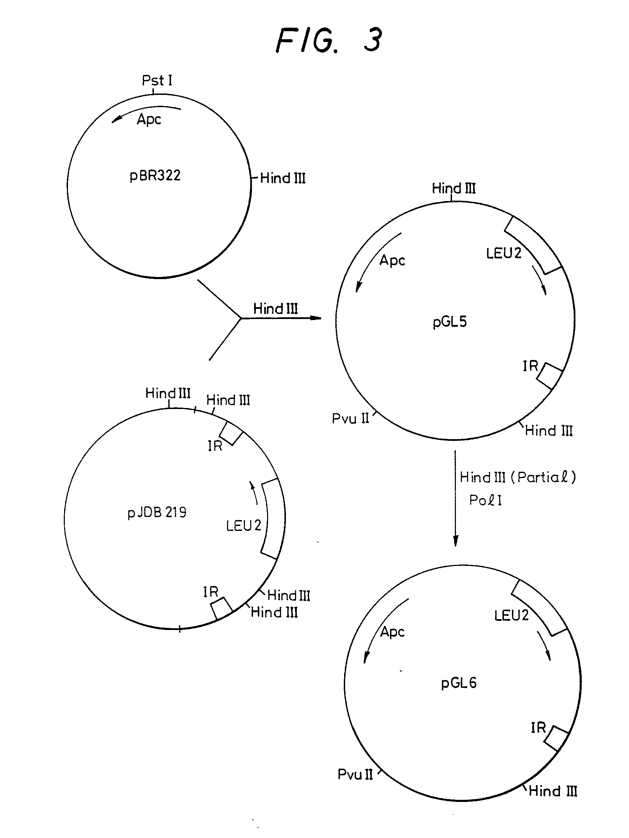

A plasmid vector, pGG5, using the GAP-DH promoter for the expression of HBsAg (hepatitis B virus surface antigen) in yeast was constructed as illustrated in Fig. 2 and Fig. 3. Referring to these figures, the construction of pGG5 is described below.

The DNA fragment 3 was prepared by complete digestion of 4 $\mu$g of the pGAP301 DNA with 1 U of TaqI (NEB) followed by electrophoretic isolation. This fragment was derived from the region of the GAP-DH promoter 2 and contained 652 bp from -676 to -25 with the base of the translation initiation site of the GAP-DH gene 1 being taken as +1.

The cohesive ends of this DNA fragment 3 was rendered blunt by treatment with 1 U of DNA polymerase I (Pol I) (Takara Shuzo) and 0.1 μg of dNTP (deoxyNTP). pUC9 [Takara Shuzo (J. Messing, Methods in Enzymology, 101 , 20 (1983))] (1 μg) was completely digested with 1 U of Sma I (Takara Shuzo) and ligated with the above blunt-ended DNA fragment in the direction shown in Figure 2 (A) using 5 U of T4 DNA ligase. The resulting DNA was used to transform Escherichia coli HB101 and the thus-obtained plasmid was named pGG2.

Then, the 140 bp DNA fragment 5 containing the terminator sequence 4 of the GAP-DH gene was prepared by completely digesting 10 μg of pGAP301 with 3 U of SalI (Takara Shuzo) and 3 U of HindIII (Takara Shuzo), followed by electrophoretic isolation. Separately, a 3.4 kbp DNA fragment was prepared by digesting 1 μg of the pGG2 DNA with 1 U of SalI (Takara Shuzo) and 1 U of HindIII, followed by electrophoretic isolation. This 3.4 kbp DNA fragment and the 140 bp DNA fragment were ligated together using 5 U of T4 DNA ligase (Takara Shuzo) and the thus-obtained plasmid was named pGG3.

DNA containing the HBsAg gene 7 was prepared by completely digesting 4 μg of pPreS (Fig. 5) with 1 U of XhoI (Takara Shuzo) and 1 U of Sal I (Takara Shuzo) and isolating the 1.3 kbp DNA fragment 6. The DNA fragment obtained by completely digesting 1 μg of the pGG3 DNA with SalI (Takara Shuzo) was ligated with the HBsAg gene-containing 1.3 kbp fragment using 5 U of T4 DNA ligase (Takara Shuzo) and the plasmid obtained was named pGG4 (Fig. 2 (B)).

For the expression of the HBsAg gene in yeast, it is desirable that the HBsAg gene should be present on a self-reproducing DNA. However, pGG4 cannot reproduce itself in yeast. Therefore, 5 μg of the Escherichia coli-yeast shuttle vector pJDB219 DNA (Beggs, J.D., Nature, 275 :104 (1978)) was completely digested with HindIII. A 3.2 kbp DNA fragment was thus prepared. The Escherichia coli plasmid pBR322 DNA (1 μg) was completely digested with Hin dIII and ligated with the above 3.2 kbp DNA fragment using 5 U of T4 DNA ligase. Thus was constructed the shuttle vector pGL5 having two HindIII sites. One of the two HindIII sites of pGL5 was rendered blunt-ended using DNA polymerase (Takara Shuzo), whereby the plasmid pGL6 having only one HindIII site (Fig. 3).

pGL5 has the yeast 2 μ DNA-derived replication origin and the LEU2 gene (yeast-derived marker gene) on the 3.2 kbp HindIII DNA fragment and, on the other fragment, it has the replication origin of Escherichia coli and the ampicillin resistance gene (Apc), which is a marker gene for Escherichia coli. To obtain the yeast replication origin and the LEU2 gene, 2 μg of the pGL5 DNA was completely digested with 1 U of HindIII and then the 3.2 kbp DNA was isolated by electrophoresis. Then, pGG4 DNA (1 μg) was completely digested with 1 U of HindIII and ligated with the 3.2 kbp DNA fragment using 5 U of T4 DNA ligase. The plasmid pGG5 was thus constructed (Fig. 2 (B)). In this way, the vector pGG5 (Fig. 2 (B)) for the production of HBsAg in yeast, which contains the promoter region having a sufficient length (652 bp) for its functioning as the GAP-DH promoter, and the HBsAg gene and the GAP-DH terminator is constructed.

## REFERENCE EXAMPLE 3

### Miniaturization of the GAP-DH promoter region

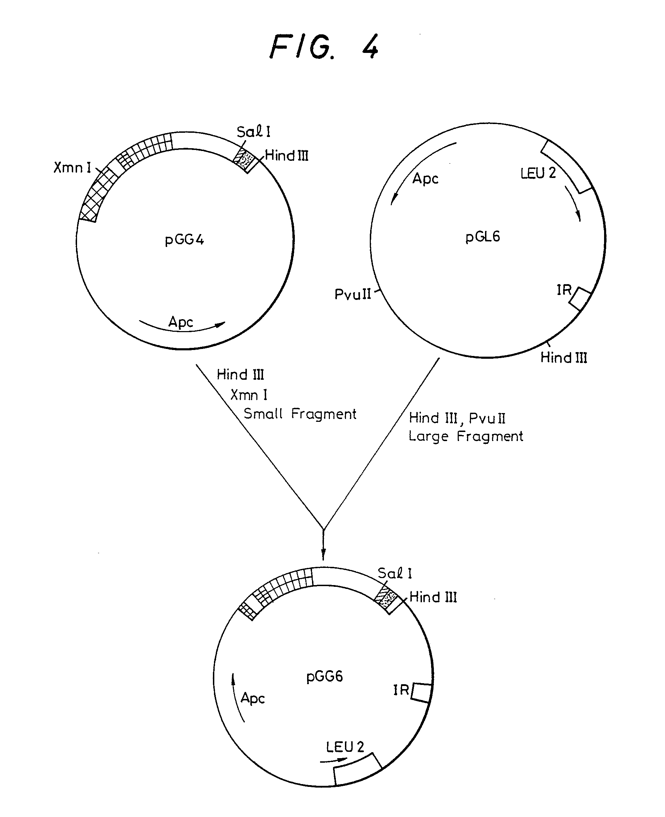

Since the site of the promoter region for the GAP-DH gene is unknown, promoter region miniaturization was attempted using various restriction enzymes. A 1.8 kbp DNA fragment containing the GAP-DH promoter (-165 bp to -25 bp), HBsAg gene and GAP terminator was prepared by completely digesting 2 μg of pGG4 with 1 U of the restriction enzyme XmnI (NEB) and 1 U of HindIII (Takara Shuzo), followed by electrophoretic isolation. Separately, a 5.6 kbp DNA fragment was prepared by completely digesting 1 μg of pGL6 with 1 U of PvuII (Takara Shuzo) and 1 U of HindIII Takara Shuzo). The 1.8 kbp DNA fragment and the 5.6 kbp DNA fragment were ligated together using T4 DNA ligase (Takara Shuzo) and the thus-constructed plasmid containing the GAP-DH promoter shown in Fig. 1 which was named pGG6 was obtained (Fig. 4).

The yeast Saccharomyces cerevisiae GRF18 (α, his, leu, trp, met) strain was transformed using either pGG6 or pGG5. The transformant obtained was cultured on a leucine-free minimal medium plate comprising 0.7% (w/v) Yeast Nitrogen Base (Difco), 2% (w/v) dextrose, 1.5% (w/v) agar. The purified Saccharomyces cerevisiae strain GRF18/pGG6 was shake-cultured on the above minimal medium (but free of agar) at 30°C for 2 days. The culture was used as a pre culture and inoculated into 80 ml of the same minimal medium to a concentration of 1% (w/v). After cultivation at 30°C for 2 days, cells were harvested by centrifugation and washed once with physiological saline. A 1-mg portion of the cells was added to 16 ml of a buffer solution comprising 50 mM Tris-HCl, pH 7.5, 1.0 mM EDTA, and caused to be suspended therein. The buffer

7

suspension was sonicated at level 10 on a Tomy Seiko model UR-200p sonicator for 9 minutes with ice cooling and then centrifuged at 0°C and 13,000 x g for 10 minutes. The supernatant thus obtained was assayed for HBsAg activity by RPHA using Antihebcel(R)(Green Cross Corp.). The results are shown in Table 1 below.

## Table 1

| Plasmid | Host | HBsAg activity ($2^n$) [*] |
|---------|------|------------------------|
| pGG5 | Saccharomyces cerevisiae GRF18 | 8 |
| pGG6 | Same as above | 8 |

*n indicates the number of dilutions.

From the results shown in Table 1, it can be seen that the HBsAg activity of Pre S-HBsAg produced by the yeast transformed with a plasmid containing the full length GAP-DH promoter sequence (pGG5) and that of Pre S-HBsAg produced by the yeast transformed with a plasmid containing a miniaturized upstream-deleted GAP-DH promoter sequence (pGG6) are equivalent.

## REFERENCE EXAMPLE 4

### Construction of Pre S-HBsAg expression vector

A vector was obtained by completely digesting pGG5 with 2 U of BamHI (Takara Shuzo) and isolating a 7 kbp DNA fragment by electrophoresis.

pPreS (Fig. 5) was completely digested with 2 U of MstII (NEB) and 2 U of BanII (NEB) and a 1.4 kbp DNA fragment was prepared by electrophoresis. This DNA fragment codes for Pre S-HBsAg composed of HBsAg and a polyalbumin receptor-containing 55-amino-acid residue polypeptide joined thereto on the N terminus side thereof. These two DNA fragments were respectively treated with 2 U of DNA polymerase (Takara Shuzo) and dNTP (deoxyNTP) to thereby render the cohesive ends blunt. These DNA fragments were then ligated together using 5 U of T4 DNA ligase (Takara Shuzo). The resulting DNA was used to transform Escherichia coli HB101. The thus-obtained plasmid was named pGG53 (Fig. 5).

## EXAMPLE 1

A vector was prepared by completely digesting pGG6 carrying the miniaturized GAP-DH promoter and the HBsAg gene with 2 U of BamHI (Takara Shuzo) and isolating a 6 kbp DNA fragment by electrophoresis.

Separately, a 1.4 kbp DNA fragment was prepared by completely digesting pPreS carrying the Pre S gene 8 and the HBsAg gene with 2 U of MstII (NEB) and 2 U of BanII (NEB), followed by electrophoresis. This DNA fragment codes for Pre S-HBsAg.

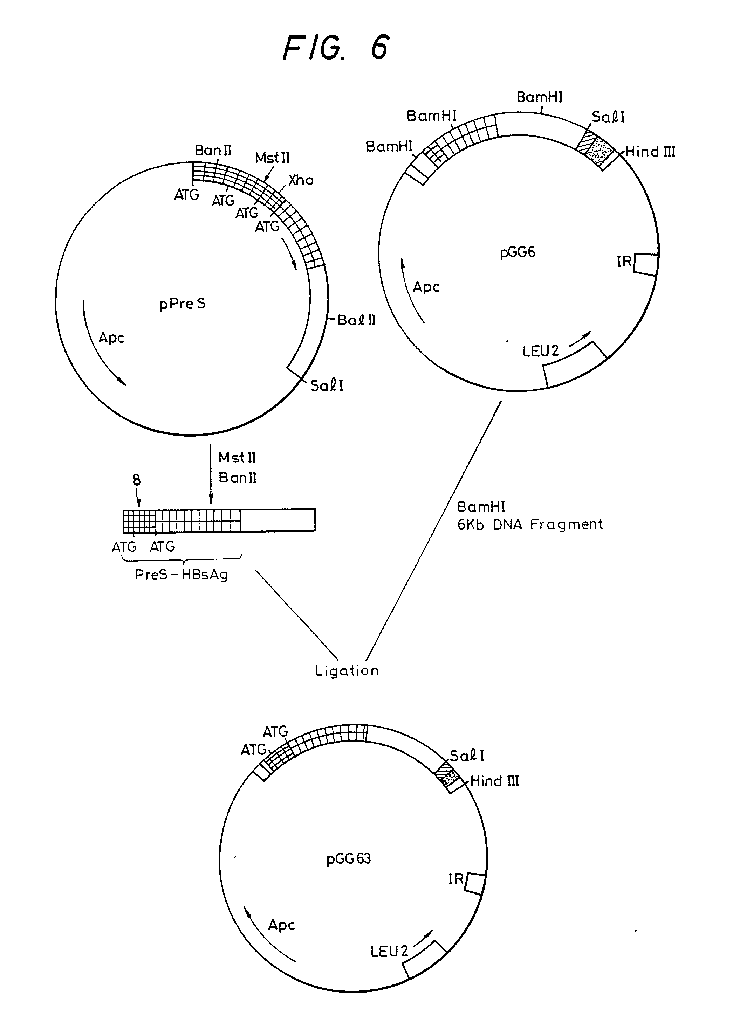

The above two DNA fragments were respectively treated with 2 U of DNA polymerase (Takara Shuzo) and dNTP (deoxyNTP) to thereby render the cohesive ends blunt. These DNA fragments were ligated together using 5 U of T4 DNA ligase (Takara Shuzo). The plasmid obtained as a result of transformation of Escherichia coli HB101 with the resultant DNA was named pGG63 (Fig. 6).

Then, yeast Saccharomyces cerevisiae AH22 (a, his4, leu2, can1) was transformed with pGG63 or pGG53 and each transformant obtained was cultured on a minimal medium plate comprising 0.7% (w/v) Yeast Nigrogen Base (Difco), 2% (w/v) dextrose, 1.5% (w/v) agar. The thus-purified transformants Saccharomyces cerevisiae AH22/pGG63 and Saccharomyces cerevisiae AH22/pGG53 were each shake-cultured on the above minimal medium (but free of agar) at 30°C for 2 days. The culture was used as a preculture and inoculated into 80 ml of the same minimal medium to a concentration of 1% (w/v). After cultivation at 30°C for 2 days, cells were collected by centrifugation and washed once with physiological saline. A 1-mg portion of the cells was added to 16 ml of a buffer comprising 50 mM Tris-HCl, pH 7.5, 1.0 mM EDTA, 1.0 mM PMSF, and caused to be suspended therein. The buffer suspension was treated on a Tomy Seiko model UR-200p sonicator at level 10 for 9 minutes with ice cooling and then subjected to centrifugation at 0°C and 13,000 x g for 10 minutes. The supernatant was assayed for HBsAg activity using Antihebcel[R] (Green Cross Corp.) and for polyalbumin receptor activity using an RPHA reagent obtained by sensitization of ovine erythrocytes with polyalbumin.

Polyalbumin-sensitized erythrocytes were prepared as follows. 100 mg of human serum albumin (Miles Scientific) was dissolved in 9 ml of 0.1 M phosphate buffer (pH 6.8), and 1 ml of 2.5% aqueous glutaraldehyde solution was added to the solution to prepare a mixture.

The mixture was placed in a test tube and stirred gently at 20°C and at a rotation speed of 60 rpm for 3 hours using a slant rotar (Daiichi Radioisotope).

The mixture was transferred into a dialysis tube and dialyzed for 3 hours with exchanging 0.01 M phosphate buffer saline (PBS) at pH 7.2 frequently to remove excessive glutaraldehyde.

The glutaraldehyde-treated polyalbumin in the dialysis tube was introduced in Sephadex G-200 column (1.5 cm x 85 cm) equated with 0.2 M Tris-HCl buffered 0.2 M saline (pH 8.0 ) (TBS) and fractionated to obtain 5 ml fractions.

Optical density at 280 nm of each fraction was measured and the fraction corresponding to the first peak was introduced in a dialysis tube and dialyzed against PBS sufficiently to prepare polyalbumin.

4 ml of 10% suspension of ovine erythrocytes and 4 ml of polyalbumin (30 mg/ml) were mixed and 2 ml of 2.5% glutaraldehyde was portionwise added to the mixture, which was then allowed to stand at room temperature for 2 hours with gentle stirring. After washing with PBS three times the product was suspended in PBS containing 1% rabbit serum and 1% sucrose (Nakarai Kagaku). The resulting suspension was used as polyalbumin-sensitized erythrocyte.

The results are shown in Table 2 below.

### Table 2

| Plasmid | Host | HBsAg activity $(2^n)^*$ | Polyalbumin receptor activity $(2^n)^*$ |
|---------|------|--------------------------|------------------------------------------|
| pGG63 | Saccharomyces cerevisiae AH22 | 7 | 6 |
| pGG53 | Same as above | 7 | 6 |

\* n indicates the number of dilutions.

From the results shown in Table 2, it can be seen that the HBsAg activity and polyalbumin receptor activity of Pre S-HBsAg produced by the yeast transformed with a plasmid containing the full length GAP-DH promoter sequence (pGG53) are equivalent to the activities of Pre S-HBsAg produced by the yeast transformed with a plasmid containing a miniaturized upsteam-deleted GAP-DH promoter (pGG63). From this it follows that the miniaturized GAP-DH promoter exhibits a promoter activity as potent as the full length GAP-DH promoter in the expression of Pre S-HBsAg consisting of the late part of the Pre S region beginning with the third ATG site.

The supernatant from yeast transformed with pGG63 was purified by centrifugation on a cesium chloride density gradient. The molecular weight of the purified sample was determined by SDS PAGE was about 30,000 ± 2,000. The production of Pre S-HBsAg in the yeast transformant, carrying pGG63 was thus confirmed. It was also confirmed that the HBsAg particles obtained had a particle size of about 22 nm as determined by electron microscopy.

### EXAMPLE 2

The plasmid pGG6 carrying the miniaturized GAP-DH promoter and the HBsAg gene was completely digested with 2 U of BamHI (Takara Shuzo) and a 6 kbp DNA fragment was prepared by electrophoresis. Thus was obtained a vector.

The plasmid pPreS carrying the Pre S gene 8 was completely digested with 2 U of BanII (NEB) and a 1.5 kbp DNA fragment was prepared by electrophoresis. This DNA fragment codes for Pre S-HBsAg.

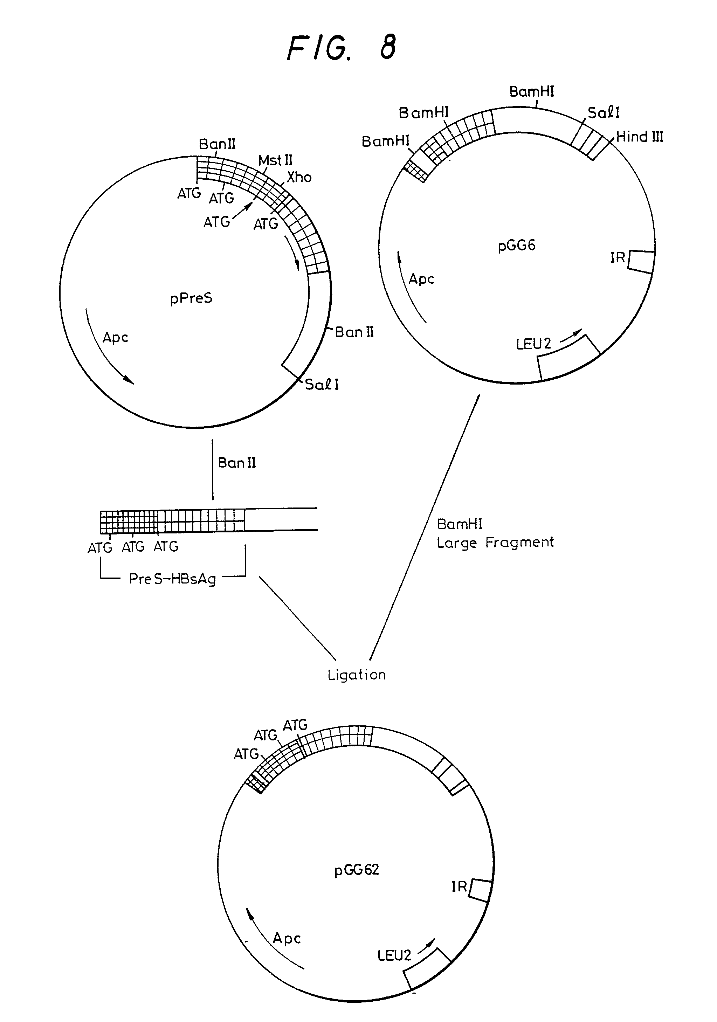

The above two DNA fragments were each treated with 2 U of DNA polymerase (Takara Shuzo) and dNTP (deoxyNTP) to thereby render the cohesive ends blunt. These DNA fragments were ligated together using 5 U of T4 DNA ligase (Takara Shuzo). The resultant DNA was used to transform Escherichia coli HB101 and the plasmid thus obtained was named pGG62 (Fig. 8).

Then, the yeast Saccharomyces cerevisiae AH22 (a, his4, leu2, can1) was transformed with pGG62 or pGG53. The transformant obtained was cultured on a leucine-free minimal medium plate comprising 0.7% (w/v) Yeast Nigrogen Base (Difco), 2% (w/v) dextrose, 1.5% (w/v) agar. The thus-purified transformants Saccharomyces cerevisiae AH22/pGG62 and Saccharomyces cerevisiae AH22/pGG53 were each shake-cultured on the above minimal medium (but free of agar) at 30°C for 2 days. The culture was inoculated, as a preculture, into 80 ml of the same minimal medium to a concentration of 1% (w/v). After 2 days of cultivation at 30°C, cells were harvested by centrifugation, washed once with physiological saline and suspended in a buffer comprising 50 mM Tris-HCl, pH 7.5, 1.0 mM EDTA, 1.0 mM PMSF. The suspension was treated on a Tomy Seiko model UR-200p sonicator at level 10 for 9 minutes with ice cooling and then subjected to centrifugation at 0°C and 13,000 x g for 10 minutes. The supernatant thus obtained was assayed for HBsAg activity using Antihebcel[R] (Green Cross Corp.) and for polyalbumin receptor activity using an RPHA reagent obtained by sensitization of ovine erythrocytes with polyalbumin. The results are shown in Table 3 below.

## Table 3

| Plasmid | Host | HBsAg activity $(2^n)^*$ | Polyalbumin receptor activity $(2^n)^*$ |
|---------|------|----------------|-----------------------|
| pGG62 | Saccharomyces cerevisiae AH22 | 7 | 6 |
| pGG53 | Same as above | 7 | 6 |

\* n indicates the number of dilutions.

From the results shown in Table 3, it can be seen that the HBsAg activity and polyalbumin receptor activity of Pre S-HBsAg produced by the yeast transformed with a plasmid containing the full length GAP-DH promoter sequence (pGG53) are equivalent to the activities of Pre S-HBsAg produced by the yeast transformed with a plasmid containing a miniaturized upstream-deleted GAP-DH promoter (pGG63). From this it follows that the miniaturized GAP-DH promoter exhibits a promoter activity as potent as the full length GAP-DH promoter in the expression of Pre S-HBsAg consisting of the late part of the Pre S region beginning with the second ATG site (pGG62).

The supernatant from yeast transformed with pGG62 was purified by centrifugation on a cesium chloride density gradient. The molecular weight of the purified sample was determined by SDS PAGE and found to be about 32,000 ± 2,000. It was thus confirmed that the yeast strain transformed with pGG62 had produced Pre S-HBsAg.

While the invention has been described in detail and with reference to specific embodiments thereof, it will be apparent to one skilled in the art that various changes and modifications can be made therein without departing from the spirit and scope thereof.

## Claims

1. A method of producing Pre S-HBsAg comprising

(A) culturing Saccharomyces cerevisiae which has been transformed with an expression vector comprising pBR322 having inserted therein sequentially, a substantial part of the 164-base-pair region of the yeast glyceraldehyde-3-phosphate dehydrogenase (GAP-DH) promoter upstream from the initiation codon for the GAP-DH portion, and the Pre s-HBsAg gene containing at least a late part of the Pre S region of HB virus, wherein said substantial part of the 164-base-pair region beginning with the -164th base pair upstream from said initiation codon and

(B) obtaining Pre S-HBsAg from the culture supernatant.

2. The method of producing Pre S-HBsAg as claimed in claim 1, wherein said substantial part comprises at least the base sequence from -164 bp to -25 bp upstream from the initiation codon for the GAP-DH promoter.

3. The method of producing Pre S-HBsAg as claimed in claim 1, wherein the GAP-DH promoter has the sequence:

$$
\begin{array}{cc}
-160 & -140 \\
\text{TATTCCCCTACTTGACTAATAAGTA} &
\end{array}
$$

$$
\begin{array}{cc}
-130 & -110 \\
\text{TATAAAGACGGTAGGTATTGATTGTAATTC} &
\end{array}
$$

$$
\begin{array}{cc}
-100 & -80 \\
\text{TGTAAATCTATTTCTTAAACTTCTTAAATT} &
\end{array}
$$

$$
\begin{array}{cc}
-70 & -50 \\
\text{CTACTTTTATAGTTAGTCTTTTTTTTAGTT} &
\end{array}
$$

$$
\begin{array}{c}
-40 \\
\text{TTAAAACACCAAGAACTTAGTTTCG}
\end{array}
$$

4. The method of producing Pre S-HBsAg as claimed in claim 1, wherein the late part of the Pre S region begins with the second or third ATG site in said region.

5. The method of producing Pre S0-HBsAg as claimed in claim 4, wherein the polypeptide portion encoded by said late part of the Pre S region comprises 55 amino acid residues and has the sequence:

Met His Trp Asn Ser Thr Thr Phe His Gln Thr
ATG CAC TGG AAC TCC ACA ACC TTC CAC CAA ACT
Leu Gln Asp Pro Arg Val Arg Gly Leu Tyr Phe
CTG CAA GAT CCC AGA GTG AGA GGC CTG TAT TTC
Pro Ala Gly Gly Ser Ser Ser Gly Thr Val Asn
CCT GCT GGT GGC TCC AGT TCA GGG ACA GTA AAC
Pro Val Pro Thr Thr Thr Ser Pro Ile Ser Ser
CCT GTT CCG ACT ACT ACC TCT CCC ATA TCG TCA
Ile Phe Ser Arg Ile Gly Asp Pro Ala Leu Asn
ATC TTC TCG AGG ATT GGG GAC CCT GCG CTG AAC

6. The method of producing Pre S-HBsAg as claimed in any of claims 1 to 5, wherein the HBsAg is of the subtype ad/yw.

7. The method of producing Pre S-HBsAg as claimed in any of claims 1 to 5, wherein the HBsAg is of the subtype adw or ayw.

8. The method of producing Pre S-HBsAg as claimed in any of claims 1 to 5, wherein said Saccharomyces cerevisiae is strain AH22.

9. The Pre S-HBsAg produced by the method of claim 1.

0 248 167

# FIG. 1

-150                                                                -101

TATTCCCCTACTTGACTAATAAGTATATAAAGACGGTAGGTATTGATTGTAATTCTGTAAATCT

-100                                                           -50

ATTTCTTAAACTTCTTAAATTCTACTTTTATAGTTAGTCTTTTTTTTAGTTTTAAAACACCAAGAACTTAGTTTCG

# FIG. 2(A)

# FIG. 2(B)

# FIG. 3

# FIG. 4

# FIG. 5

# FIG. 6

0 248 167

## FIG. 7

# FIG. 8